# EUROPEAN PATENT SPECIFICATION

(11) **EP 0 389 180 B1**
(45) Date of publication and mention of the grant of the patent: **04.01.1995**
(21) Application number: 90302760.5
(22) Date of filing: 15.03.1990
(51) Int. Cl.: C07K 7/06, A61K 37/02, C07K 7/26

(54) **Peptides and their use in therapy**
Peptide und deren Benützung in der Therapie
Peptides et leur emploi en thérapie

(30) Priority: 15.03.1989 US 323777
(43) Date of publication of application: 26.09.1990
(73) Proprietor: BIOMEASURE, INC., Hopkinton Massachusetts 01748 (US)
(72) Inventor: Eck, Charles R., Shrewsbury, Massachusetts 01545 (US); Moreau, Sylvianne, Upton, Massachusetts 01568 (US)
(74) Representative: Deans, Michael John Percy

(56) References cited:
- EP-A- 0 122 712
- EP-A- 0 201 260
- EP-A- 0 215 171
- EP-A- 0 225 746
- J. CHEM. SOC. PERKIN TRANS. I, 1986, pages 989-1003; M.C. ALLEN et al.:
- J. CHEM. SOC. PERKIN TRANS. I, 1981, pages 2040-2048; M.C. ALLEN et al.:"Tritiated peptides. Part 11. Synthesis of [4-3H-Phe6]-, [4-3H-Phe11]-, and [4-3H-Phe6,11]-Somatostatin and the metabolite [des-Ala1]-Somatostatin"
- PEPTIDES, vol. 7, 1986, pages 953-959, Ankho International Inc., US; M. ZEGGARIet al.: "Characterization of pancreatic somatostatin binding sites with a 125I-Somatostain 28 analog"
- BIOCHEMISTRY, vol. 27, 1988, pages 1425-1432, American Chemical Society; K.NIKOLICS et al.: "Photoaffinity labeling of pituitary GnRH receptors:Significance of the position of photolabel on the ligand"
- CHEMICAL ABSTRACTS, vol. 94, 1981, page 772, abstract no. 175498m, Columbus,Ohio, US; H.J. WIENEKE et al.: "Towards the synthesis of [A-19]13-iodo-, and 3,5-diiodotyrosine porcine insulins", & PEPT., STRUCT. BIOL. FUNCT.,PROC. AM. PEPT. SYMP., 6TH 1979, 515-18

## Description

This invention relates to therapeutic peptides.

A number of somatostatin analogs exhibiting GH-release-inhibiting activity have been described in the literature, including analogs containing fewer than the naturally occurring fourteen amino acids. For example, Coy et al. U.S. Patent No. 4,485,101, the disclosure of which is to be regarded as hereby incorporated by reference, describe dodecapeptides having an N-terminal acetyl group, a C-terminal NH₂, D-Trp at position 6, and p-C1-Phe at position 4. (Herein, when no designation of configuration is given, the L-isomer is intended).

EP-A-0215171 discloses a number of somatostatin analogues, all being octapeptides having D-Trp at position 4 and optional modifications at positions 3 and 6.

In general, the invention features in one aspect thereof an octapeptide of the formula: 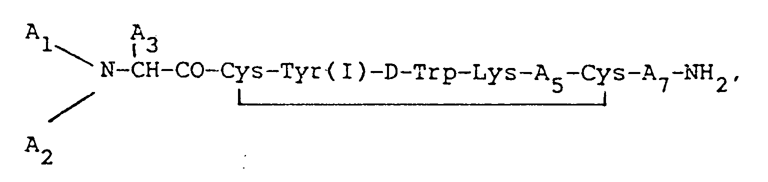
wherein each A₁ and A₂, independently, is H, C₁₋₁₂ alkyl, C₇₋₁₀ phenylalkyl, R₁CO (where R₁ is C₁₋₂₀ alkyl, C₃₋₂₀ alkenyl C₃₋₂₀ alkynyl, phenyl, naphthyl, or C₇₋₁₀ phenylalkyl), or R₂OCO (where R₂ is C₁₋₁₀ alkyl or C₇₋₁₀ phenylalkyl), provided that when one of A₁ or A₂ is R₁CO or R₂OCO, the other must be H; A₃ is CH₂-A₆ (where A₆ is pentafluorophenyl, naphthyl, pyridyl, phenyl, or o- m-, or, more preferably, p-substituted phenyl, where the Substituent is a halogen, NH₂, NO₂, OH, or C₁₋₃ alkyl); (I) indicates that tyrosine is iodinated on the phenyl ring at its 3 or 5 carbon positions; A₅ is Thr, Ser, Phe, Val, α-aminobutyric acid, or Ile, provided that when A₃ is phenyl, A₁ is H, and A₂ is H, A₅ cannot be Val; and A₇ is Thr, Trp, or β-Nal; or a pharmaceutically acceptable salt thereof.

In the formula given above, the configuration of the molecule at the carbon atom to which A₃ is bonded is not given, to indicate that the amino acid residue of which A₃ is a substituent can have the D- or L- configuration. In the formula given above, there is a bond shown between the two Cys residues to indicate cyclization.

By 3 0r 5 positions is meant carbon atoms in the ortho position relative to the -OH group of tyrosine.

Preferred compounds of this formula include D-β-Nal-Cys-Tyr(I)-D-Trp-Lys-Val-Cys-Thr-NH2
(also termed [I]-BIM-23014C);

D-Phe-Cys-Tyr(I)-D-Trp-Lys-α-
Aminobutyric acid-Cys-Thr-NH2; pentafluoro-D-Phe-Cys-
Tyr(I)-D-Trp-Lys-Val-Cys-Thr-NH2; N-Ac-D-β-Nal-Cys-
Tyr(I)-D-Trp- Lys-Val-Cys-Thr-NH2; D-β-Nal-Cys-Tyr(I)-
D-Trp-Lys-Val-Cys-β-Nal-NH2; D-Phe-Cys-Tyr-(I)-D-Trp-
Lys-Val-Cys-β-Nal-NH2; D-β-Nal-Cys-Tyr(I)-D-Trp-Lys-α-
aminobutyric acid-Cys-Thr-NH2; D-p-Cl-Phe-Cys-Tyr(I)-
D-Trp-Lys-α-aminobutyric acid-Cys-Thr-NH2; and acetyl-
D-p-Cl-Phe-Cys-Tyr(I)-D-Trp-Lys-α-aminobutyric acid-
Cys-Thr-NH2.

In an alternative aspect, the invention features an octapeptide having a biological activity of somatostatin, having at amino acid position 3 a tyrosine residue, the tyrosine having an iodine atom at its 3 or 5 carbon position, or a pharmaceutically acceptable salt of said octapeptide. By biological activity of somatostatin is meant a polypeptide exhibiting GH-releasing-inhibiting activity.

In preferred embodiments, a therapeutically effective amount of the compound is admixed with a pharmaceutically acceptable carrier substance (e.g. magnesium carbonate, lactose, or a phospholipid with which the therapeutic compound can form a micelle). The most preferred carrier substance is mannitol. Examples of such compositions include a pill, tablet, capsule, or liquid for oral administration to a human patient, a spreadable cream, gel, lotion, or ointment for application to the skin of a human patient in need of the compound, a liquid capable of being administered nasally as drops or spray, or a liquid capable of intravenous, parenteral, subcutaneous, or intraperitoneal administration. The pill, tablet or capsule can be coated with a substance capable of protecting the composition from the gastric acid in the patient's stomach for a period of time sufficient to allow the composition to pass undisintegrated into the patient's small intestine. The therapeutic composition can also be administered in the form of an oil emulsion or dispersion in conjunction with a lipophillic salt such as a pamoic acid. The therapeutic composition can also be in the form of a biodegradable sustained release formulation for intramuscular administration. For maximum efficacy, zero order release is desired. Zero order release can be obtained using an implantable or external pump, e.g., Infusaid ™ pump, to administer the therapeutic composition.

We have found our compounds to be active in inhibiting primarily the secretion of GH, and to a lesser extent that of insulin, and glucagon. Further, the aromatic lipophilic N-terminal end can provide long-lasting in vivo activity. Provision of iodine in the third amino acid increases the metabolic stability of the somatostatin octapeptide analog. Such analogs are thus longer acting GH suppressants.

Other features and advantages of the invention will be apparent from the following description by way of example only of preferred embodiments.

Our compounds of formula: 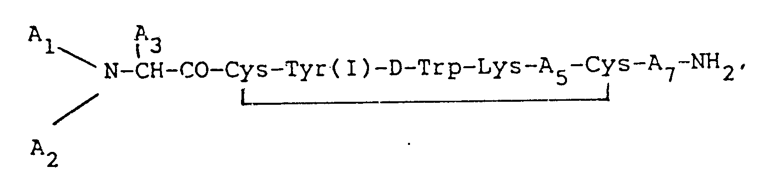
where A₁, A₂, A₃, A₅ and A₇ are each as defined above, are all octapeptide analogs of somatostatin which have 3- or 5-iodo-tyrosine at position 3; D-Trp at position 4; and optional modifications at positions 6 (A₅), and 8(A₇).

Alternatively, our compounds may be seen as polypeptides having somatostatin-like activity. We have found that D-β-naphthylalanine at position 1, and Tyr at position 3, modified to have an iodine atom at carbon position 3 or 5, and Val at position 6, are modifications which particularly enhance activity and stability.

The compounds can be provided in the form of pharmaceutically acceptable salts or complexes. Examples of preferred salts or complexes are those with therapeutically acceptable organic acids, e.g., acetic, lactic, maleic, citric, malic, ascorbic, succinic, benzoic, salicylic, methanesulfonic, toluenesulfonic, or pamoic acid, as well as polymeric acids such as tannic acid or carboxymethyl cellulose, and salts with inorganic acids such as the hydrohalic acids, e.g., hydrochloric acid, sulfuric acid, or phosphoric acid.

Examples of synthesis of octapeptides in accordance with this invention are given bleow. Generally, the octapeptide is first synthesized and then iodinated by any of several procedures to provide an iodinated tyrosine at position 3.

The synthesis of one octapeptide follows. Other octapeptides and somatostatin-like compounds in accordance with this invention can be prepared by making appropriate modifications, within the ability of someone of ordinary skill in this field, of the following synthetic method.

The first step in the preparation of D-β-naphthylalanine-Cys-Tyr(I)-D-Trp-Lys-Val-Cys-Thr-NH₂ was the preparation of the intermediate tert-butyloxycarbonyl-D-β-napthylalanine-S-methylbenzyl-Cys-Tyr-D-Trp-N^{ε}-benzyloxycarbonyl-Lys-Val-S-methylbenzyl-Cys-O-benzyl-Thr-benzyhydrylamine resin, as follows.

Benzhydrylamine-polystyrene resin (Vega Biochemicals, Inc.) in the chloride ion form was placed in the reaction vessel of a Beckman 990B peptide synthesizer programmed to perform the following reaction cycle: (a) methylene chloride; (b) 33% trifluoroacetic acid in methylene chloride (2 times for 1 and 25 min each); (c) methylene chloride; (d) ethanol; (e) methylene chloride; (f) 10% triethylamine in chloroform.

The neutralized resin was stirred with Boc-O-benzyl-threonine and diisopropylcarbodiimide (1.5 mmole each) in methylene chloride for 1 h and the resulting amino acid resin was then cycled through steps (a) to (g) in the above wash program. The following amino acids (1.5 mmole) were then coupled successively by the same procedure: Boc-S-methylbenzyl-Cys, Boc-Val, Boc-Ne-benzyloxycarbonyl-lysine, Boc-D-Trp, Boc-Tyr, Boc-S-methylbenzyl-Cys, Boc-D-β-naphthylalanine.

The resin was washed and dried and then mixed with anisole (4 ml) and anhydrous hydrogen fluoride (36 ml) at 0°C and stirred for 45 min. (one can also use thioanisole, trifluoroacetic acid, and trifluoromethane sulfonic acid at a ratio of 1:90:9, for 6h). Excess hydrogen fluoride was evaporated rapidly under a stream of dry nitrogen and free peptide precipitated and washed with ether. The crude peptide was then dissolved in 800 ml of 90% acetic acid to which was added I₂ in methanol until a permanent brown color was present. The solution was then stirred for 1 h before removing the solvent in vacuo. The resulting oil was dissolved in a minimum volume of 50% acetic acid and eluted on a column (2.5 X 100 mm) of Sephadex G-25. Fractions containing a major component by uv absorption and thin layer chromatography were then pooled, evaporated to a small volume, and applied to a column (2.5 X 50 cm) of Whatman LRP-l octadecylsilane (15-20 uM).

The column was eluted with a linear gradient of 10-50% acetonitrile in 0.1% trifluoroacetic acid in water. Fractions were examined by thin layer chromatography and analytical high performance liquid chromatography and pooled to give maximum purity and if desired, a different salt prepared, e.g., acetate or phosphate. Repeated lyophilization of the solution from water gave 170 mg of the product as a white, fluffy powder.

The product was found to be homogeneous by Hplc and Tlc. Amino acid analysis of an acid hydrolysate confirmed the composition of the octapeptide. The octapeptide was then iodinated as follows.

The following methods are examples of methods which can be used to iodinate the above somatostatin analogues which contain the amino acid tyrosine at position 3. For example, iodination may be by use of: a) Chloramine-T/NaI; b) Lactoperoxidase-glucose oxidase (LP-GO)/NaI; c) Lactoperoxidase-H₂O₂ (LP-H₂O₂)/NaI; d) Enzymobeads (immobilized LP-GO)/NaI (purchased from Bio-Rad Laboratories); e) Iodobeads (immobilized chloromine-T)NaI (purchased from Pierce Chemical Company); f) Iodogen/NaI; g) Iodine/KI; and h) Iodine monochloride.

After iodination, the iodinated products are purified, e.g., by HPLC to separate di-iodination products of tyrosine, and unreacted starting material, from the desired iodinated octapeptide. The following is a specific example showing Chloramine-T/NaI iodination of the octapeptide BIM-23014C.

250mg of BIM-23014C (0.22 mmole) was placed in a 500ml Erlenmeyer flask, and 67.3ml (67.3mg, 0.44mmole) of sodium iodide in 0.05M phosphate buffer, pH7.4, added. An additional 160ml of 0.05M phosphate buffer, pH7.4, was then added to the reaction vessel and stirred until all the peptide dissolved. 150ml (0.17mmole) chloramine-T dissolved in phosphate buffer, pH7.4, was added to the reaction vessel, and swirled continuously for 3 minutes. The reaction was stopped by addition of 20ml 0.014M sodium thiosulfate solution. Finally, 1.5ml of glacial acetic acid was added, and the solution filtered.

For purification the solution (∼250ml) was injected into a preparative HPLC column and eluted with a gradient as follows. The HPLC instrument was a SepTech 800ST; and the column was a Vydac 15-20µ C₁₈ column. The mobile phase consisted of A (0.05M NH₄OAc, ph 4.5); and B (0.05M NH₄OAc, pH 4.5, and CH₃CN, at a ratio of 1:1) with the gradient constructed from 30%B to 75%B over 45 minutes, and then 75%B for 10 minutes, at a flow rate of about 30ml/min. Progress was followed using a λ of 228nm. Six fractions were collected, and some assayed for octapeptide content by HPLC. Those with the majority of the desired octapeptide were combined and evaporated to a lower volume (∼80ml) by rotary evaporation under high vacuum.

The resulting liquid was reloaded onto the preparative HPLC column for a final purification using conditions as before. Six fractions were collected and assayed for content by HPLC. One fraction contained the octapeptide and was concentrated to about 100ml by rotary evaporation, transferred to a 500ml Buchner flask, frozen, and lyophilized for 24 hours. The sample was redissolved in 50ml of H₂O and relyophilized for 48 hours. The final product (190mg), a fluffy white powder, was transferred to a labelled sample storage bottle, capped and stored at -20°C. The product is [I]-BIM-23014C.

1.2mg of [I]-BIM-23014c was dissolved in 1.0ml of saline and assayed for purity by HPLC (mobile phase was 0.05M NH₄OAc, pH 4.6 and CH₃CN, at a ratio of 6:4; flow rate was 1.0ml/min., progress was followed at a λ of 228nm, on 5µC₁₈ Nucleosil column). Triplicate 5µl injections were made and mean purity of the major peak was determined as 98.8%. The major impurity of 6.8min. (1.2%) was shown to be non-iodinated BIM-23014.

Octapeptides having the formulae: 
pentafluoro-D-Phe-Cys-Tyr(I)-D-Trp-Lys-Val-
Cys-Thr-NH2, D-Phe-Cys-Tyr(I)-D-Trp-Lys-α-
aminobutyric acid-Cys-Thr-NH2, N-Ac-D-ß-Nal-Cys-Tyr(I)
-D-Trp- Lys-Val-Cys-Thr-NH2, D-ß--Nal-Cys-Tyr(I)-D-
Trp- Lys-Val-Cys-ß-Nal-NH2, D-Phe-Cys-Tyr(I)-D-Trp-Lys-
Val-Cys-ß-Nal-NH2; D-ß-Nal-Cys-Tyr(I)-D-Trp-Lys-α-
aminobutyric acid-Cys-Thr-NH2; D-p-Cl-Phe-Cys-Tyr(I)
-D-Trp-Lys-α-aminobutyric acid-Cys-Thr-NH2; and
acetyl-D-p-Cl-Phe-Cys-Tyr(I)-D-Trp-Lys-α-aminobutyric
acid-Cys- Thr-NH2
with iodine substituents in tyrosine at carbon position 3 or 5, were made according to methods analogous to those described above.

When administered to mammals, particularly humans, (e.g. orally, topically, intravenously, parenterally in a sustained release, biodegradable form, nasally, or by suppository), the compounds can be effective to inhibit GH release and to a lesser extent insulin, glucagon, and pancreatic exocrine secretion, and to therapeutically affect the central nervous system.

The compounds can be administered to a mammal, e.g. a human, in the dosages used for somatostatin or, because of their greater potency, in smaller dosages. Our compounds can be used for the treatment of cancer, particularly growth hormone-dependent cancer (e.g., bone, cartilage, pancreas (endocrine and exocrine), prostate, or breast), acromegaly and related hypersecretroy endocrine states, or of bleeding ulcers in emergency patients and in those suffering from pancreatitis or diarrhea. The compounds can also be used in the management of diabetes and to protect the liver of patients suffering from cirrhosis or hepatitis. The compounds can also be used to treat Alzheimer's disease, as analgesics to treat pain by acting-specifically on certain opiate receptors, and as gastric cytoprotective compounds for ulcer therapy. The compounds can also be used to treat certain types of mushroom poisoning.

The compounds can also be used to treat diabetes-related retinopathy. The anti-cancer activity of the compounds may be related to their ability to antagonize cancer-related growth factors such as epidermal growth factor.

In addition to the suppression of endocrine reaction the compounds can act as an antiproliferative agents by preventing the release of, or antagonizing the effects of, auto/paramine growth factor.

The compounds can be administered to a mammal, e.g., a human, in a dosage of 0.01 to 50 mg/kg/day, preferably 0.1 to 5 mg/kg/day.

## Claims (Claims for the following Contracting State(s): AT, BE, CH, DE, DK, FR, GB, IT, LI, LU, NL, SE)

1. An octapeptide having a biological activity of somatostatin, having at amino acid position 3 a tyrosine residue, said tyrosine comprising an iodine atom at its 3 or 5 carbon positions, or a pharmaceutically acceptable salt thereof.

2. An octapeptide according to Claim 1 and characterised in having the formula: 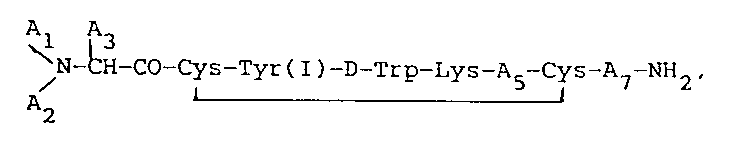 wherein each A₁ and A₂, independently, is H, C₁₋₁₂ alkyl, C₇₋₁₀phenylalkyl, R₁CO (where R₁ is C₁₋₂₀ alkyl, C₃₋₂₀ alkenyl, C₃₋₂₀ alkinyl, phenyl, naphthyl, or C₇₋₁₀ phenylalkyl), or R₂OCO (where R₂ is C₁₋₁₀ alkyl or C₇₋₁₀ phenylalkyl), provided that when one of A₁ or A₂ is R₁CO or R₂OCO, the other must be H; A₃ is CH₂-A₆ (where A₆ is pentafluorophenyl, naphthyl, pyridyl, phenyl, or o-, m-, or p-substituted phenyl, wherein said substituent is a halogen, NH₂, NO₂, OH, or C₁-C₃ alkyl); (I) indicates that tyrosine is iodinated on the phenyl ring at its 3 or 5 carbon positions; A₅ is Thr, Ser, Phe, Val, α-aminobutyric acid, or Ile, provided that when A₃ is phenyl, A₁ is H, and A₂ is H, A₅ cannot be Val; and A₇ is Thr, Trp, or β-Nal; or a pharmaceutically acceptable salt thereof.

3. An octapeptide or pharmaceutically acceptable salt thereof according to Claim 2, wherein 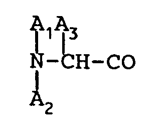 is D-β-naphthylalanine.

4. An octapeptide or a pharmaceutically acceptable salt thereof according to Claim 2, wherein 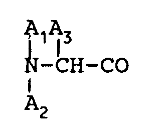 is D-Phe and A₅ is α-aminobutyric acid.

5. An octapeptide or a pharmaceutically acceptable salt thereof according to Claim 2, wherein R₁ is CH₃ or C₂H₅.

6. Octapeptides of the formulae D-β-Nal-Cys-Tyr(I)-D-Trp-Lys-Val-Cys-Thr-NH₂, pentafluoro-D-Phe-Cys-Tyr(I)-D-Trp-Lys-Val-Cys-Thr-NH₂, D-Phe-Cys-Tyr(I)-D-Trp-Lys-α-aminobutyric acid-Cys-Thr-NH₂, N-Ac-D-β-Nal-Cys-Tyr(I)-D-Trp-Lys-Val-Cys-Thr-NH₂, D-β-Nal-Cys-Tyr(I)-D-Trp-Lys-Val-Cys-β-Nal-NH₂, D-Phe-Cys-Tyr(I)-D-Trp-Lys-Val-Cys-β-Nal-NH₂, D-β-Nal-Cys-Tyr(I)-D-Trp-Lys-α-aminobutyric acid-Cys-Thr-NH₂, D-p-Cl-Phe-Cys-Tyr(I)-D-Trp-Lys-α-aminobutyric acid-Cys-Thr-NH₂, or acetyl-D-p-C1-Phe-Cys-Tyr(I)-D-Trp-Cys-α-aminobutyric acid-Cys-Thr-NH₂, or pharmaceutically acceptable salts thereof.

7. A pharmaceutical composition comprising a therapeutically effective amount of an octapeptide or a pharmaceutically acceptable salt thereof as claimed in any of Claims 1 to 6 together with a pharmaceutically acceptable carrier substance.

8. A composition according to Claim 7, wherein said composition is in the form of a pill, tablet, or capsule for oral administration to a human or animal patient, or in the form of a liquid for oral administration to a human or animal patient, or is coated with a substance capable of protecting said composition from the gastric acid in the stomach of a human or animal patient for a period of time sufficient to allow said composition to pass undisintegrated into the small intestine of said patient, or is in the form of a cream, gel, spray, or ointment for application to the skin of a human or animal patient, or is in the form of a liquid capable of being adminstered nasally as drops or spray to a human or animal patient, or is in the form of a liquid for intravenous, subcutaneous, parenteral, or intraperitioneal administration to a human or animal patient, or is in the form of a biodegradable sustained release composition for intramuscular administration to a human or animal patient.

9. An octapeptide or a pharmaceutically acceptable salt thereof according to any of Claims 1 to 6 for use in therapy for reducing or for inhibiting the release of or of antagonizing growth hormone, insulin, glucagon, or of pancreatic exocrine secretion, for the management of diabetes, to protect the livers of patients suffering from cirrhosis or hepatitis, for the treatment of Alzheimer's disease, of mushroom poisoning, or of diabetes-related retinopathy, or for the treatment of cancer, particularly growth hormone dependent cancer such as cancer of the bone, cartilage, pancreas, prostate or breast, or acromelagy and related hypersecretory endocrine states, of bleeding ulcers in emergency patients, of disorders of the central nervous system, or of patients suffering from pancreatitis or diarrhoea.

10. Use of an octapeptide or a pharmaceutically acceptable salt thereof according to any of Claims 1 to 6 for the manufacture of a medicament for use in therapy for reducing or for inhibiting the release of or of antagonizing growth hormone, insulin, glucagon, or of pancreatic exocrine secretion, for the management of diabetes, to protect the livers of patients suffering from cirrhosis or hepatitis, for the treatment of Alzheimer's disease, of mushroom poisoning, or of diabetes-related retinopathy, or for the treatment of cancer, particularly growth hormone dependent cancer such as cancer of the bone, cartilage, pancreas, prostate or breast, of acromelagy and related hypersecretory endocrine states, of bleeding ulcers in emergency patients, of disorders of the central nervous system, or of patients suffering from pancreatitis or diarrhoea.

## Claims (Claims for the following Contracting State(s): ES, GR)

1. A method for preparing a novel octapeptide comprises taking an octapeptide or a pharmaceutically acceptable salt thereof, said octapeptide having the biological activity of somatostatin, said octapeptide having a tyrosine residue at amino acid position 3; and iodinating said tyrosine on the phenyl ring at its 3 or 5 carbon position.

2. A method for preparing a novel octapeptide according to Claim 1, wherein said octapeptide has the formula: 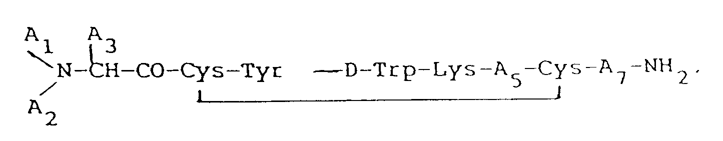 wherein each A₁ and A₂, independently, is H, C₁₋₁₂ alkyl, C₇₋₁₀phenylalkyl, R₁CO (where R₁ is C₁₋₂₀ alkyl, C₃₋₂₀ alkenyl, C₃₋₂₀ alkinyl, phenyl, naphthyl, or C₇₋₁₀ phenylalkyl), or R₂OCO (where R₂ is C₁₋₁₀ alkyl or C₇₋₁₀ phenylalkyl), provided that when one of A₁ or A₂ is R₁CO or R₂OCO, the other must be H; A₃ is CH₂-A₆ (where A₆ is pentafluorophenyl, naphthyl, pyridyl, phenyl, or o-, m-, or p-substituted phenyl, wherein said substituent is a halogen, NH₂, NO₂, OH, or C₁-C₃ alkyl); A₅ is Thr, Ser, Phe, Val, α-aminobutyric acid, or Ile, provided that when A₃ is phenyl, A₁ is H, and A₂ is H, A₅ cannot be Val; and A₇ is Thr, Trp, or β-Nal;

3. A method of preparing an octapeptide according to Claim 2, wherein 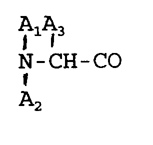 is D-β-naphthylalanine.

4. A method of preparing a novel octapeptide according to Claim 2, wherein 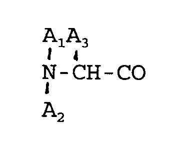 is D-Phe and A₅ is α-aminobutyric acid.

5. A method of preparing a novel octapeptide according to Claim 2, wherein R₁ is CH₃ or C₂H₅.

6. A method of preparing a novel octapeptide according to Claim 2 wherein said octapeptide is selected from: D-β-Nal-Cys-Tyr ― D-Trp-Lys-Val-Cys-Thr-NH₂, pentafluoro-D-Phe-Cys-Tyr ―D-Trp-Lys-Val-Cys-Thr-NH₂, D-Phe-Cys-Tyr ―D-Trp-Lys-α-aminobutyric acid-Cys-Thr-NH₂, N-Ac-D-β-Nal-Cys-Tyr ―D-Trp-Lys-Val-Cys-Thr-NH₂, D-β-Nal-Cys-Tyr ―D-Trp-Lys-Val-Cys-β-Nal-NH₂, D-Phe-Cys-Tyr ―D-Trp-Lys-Val-Cys-β-Nal-NH₂, D-β-Nal-Cys-Tyr ―D-Trp-Lys-α-aminobutyric acid-Cys-Thr-NH₂, D-p-Cl-Phe-Cys-Tyr ―D-Trp-Lys-α-aminobutyric acid-Cys-Thr-NH₂, or acetyl-D-p-C1-Phe-Cys-Tyr ―D-Trp-Cys-α-aminobutyric acid-Cys-Thr-NH₂,

7. A method of preparing a pharmaceutical composition comprising taking the octapeptide product of the method of any of Claims 1 to 6 and combining said product with a pharmaceutical carrier substance.

8. A method of preparing a pharmaceutical composition comprising taking an octapeptide or a pharmaceutically acceptable salt thereof, said octapeptide having the biological activity of somatostatin, having a tyrosine residue at amino acid position 3 and said tyrosine being iodinated on the phenyl ring at its 3 or 5 carbon positions, said octapeptide preferably having the formula: 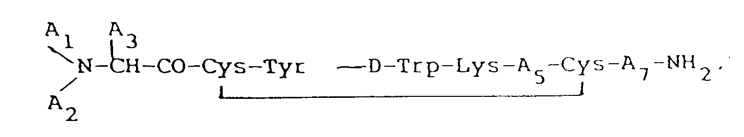 wherein each A₁ and A₂, independently, is H, C₁₋₁₂ alkyl, C₇₋₁₀phenylalkyl, R₁CO (where R₁ is C₁₋₂₀ alkyl, C₃₋₂₀ alkenyl, C₃₋₂₀ alkinyl, phenyl, naphthyl, or C₇₋₁₀ phenylalkyl), or R₂OCO (where R₂ is C₁₋₁₀ alkyl or C₇₋₁₀ phenylalkyl), provided that when one of A₁ or A₂ is R₁CO or R₂OCO, the other must be H; A₃ is CH₂-A₆ (where A₆ is pentafluorophenyl, naphthyl, pyridyl, phenyl, or o-, m-, or p-substituted phenyl, wherein said substituent is a halogen, NH₂, NO₂, OH, or C₁-C₃ alkyl); A₅ is Thr, Ser, Phe, Val, α-aminobutyric acid, or Ile, provided that when A₃ is phenyl, A₁ is H, and A₂ is H, A₅ cannot be Val; and A₇ is Thr, Trp, or β-Nal; and more preferably being selected from D-β-Nal-Cys-Tyr ― D-Trp-Lys-Val-Cys-Thr-NH₂, pentafluoro-D-Phe-Cys-Tyr ―D-Trp-Lys-Val-Cys-Thr-NH₂, D-Phe-Cys-Tyr ―D-Trp-Lys-α-aminobutyric acid-Cys-Thr-NH₂, N-Ac-D-β-Nal-Cys-Tyr ―D-Trp-Lys-Val-Cys-Thr-NH₂, D-β-Nal-Cys-Tyr ―D-Trp-Lys-Val-Cys-β-Nal-NH₂, D-Phe-Cys-Tyr ―D-Trp-Lys-Val-Cys-β-Nal-NH₂, D-β-Nal-Cys-Tyr ―D-Trp-Lys-α-aminobutyric acid-Cys-Thr-NH₂, D-p-Cl-Phe-Cys-Tyr ―D-Trp-Lys-α-aminobutyric acid-Cys-Thr-NH₂, or acetyl-D-p-C1-Phe-Cys-Tyr ―D-Trp-Cys-α-aminobutyric acid-Cys-Thr-NH₂ ; and mixing said octapeptide with a pharmaceutically acceptable carrier material to form said pharmaceutical composition.

9. Use of an octapeptide or a pharmaceutically acceptable salt thereof, said octapeptide having the biological activity of somatostatin, having a tyrosine residue at amino acid position 3 and said tyrosine being iodinated on the phenyl ring at its 3 or 5 carbon positions, said octapeptide preferably having the formula: 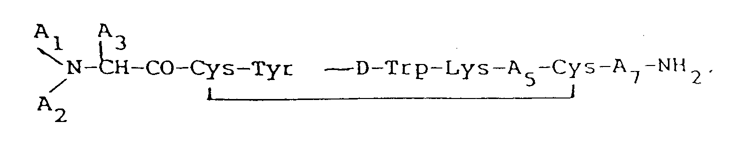 wherein each A₁ and A₂, independently, is H, C₁₋₁₂ alkyl, C₇₋₁₀phenylalkyl, R₁CO (where R₁ is C₁₋₂₀ alkyl, C₃₋₂₀ alkenyl, C₃₋₂₀ alkinyl, phenyl, naphthyl, or C₇₋₁₀ phenylalkyl), or R₂OCO (where R₂ is C₁₋₁₀ alkyl or C₇₋₁₀ phenylalkyl), provided that when one of A₁ or A₂ is R₁CO or R₂OCO, the other must be H; A₃ is CH₂-A₆ (where A₆ is pentafluorophenyl, naphthyl, pyridyl, phenyl, or o-, m-, or p-substituted phenyl, wherein said substituent is a halogen, NH₂, NO₂, OH, or C₁-C₃ alkyl); A₅ is Thr, Ser, Phe, Val, α-aminobutyric acid, or Ile, provided that when A₃ is phenyl, A₁ is H, and A₂ is H, A₅ cannot be Val; and A₇ is Thr, Trp, or β-Nal; and more preferably being selected from D-β-Nal-Cys-Tyr ― D-Trp-Lys-Val-Cys-Thr-NH₂, pentafluoro-D-Phe-Cys-Tyr ―D-Trp-Lys-Val-Cys-Thr-NH₂, D-Phe-Cys-Tyr ―D-Trp-Lys-α-aminobutyric acid-Cys-Thr-NH₂, N-Ac-D-β-Nal-Cys-Tyr ―D-Trp-Lys-Val-Cys-Thr-NH₂_{,} D-β-Nal-Cys-Tyr ―D-Trp-Lys-Val-Cys-β-Nal-NH₂, D-Phe-Cys-Tyr ―D-Trp-Lys-Val-Cys-β-Nal-NH₂, D-β-Nal-Cys-Tyr ―D-Trp-Lys-α-aminobutyric acid-Cys-Thr-NH₂, D-p-Cl-Phe-Cys-Tyr ―D-Trp-Lys-α-aminobutyric acid-Cys-Thr-NH₂, or acetyl-D-p-C1-Phe-Cys-Tyr ―D-Trp-Cys-α-aminobutyric acid-Cys-Thr-NH₂ for the manufacture of a medicament for use in therapy for reducing or inhibiting the release of or antagonising growth hormone, insulin, glucagon, or pancreatic exocrine secretion, for the management of diabetes, to protect the livers of patients suffering from cirrhosis or hepatitis, for the treatment of Alzheimer's disease, of mushroom poisoning, or of diabetes related retinopathy, or for the treatment of cancer, particularly growth hormone dependent cancer such as cancer of the bone, cartilage, pancreas, prostate or breast, of acromegaly and related hypersecretory endocrine states, of bleeding ulcers in emergency patients, of disorders of the central nervous system, or of patients suffering from pancreatitis or diarrhoea.

## Patentansprüche (Patentansprüche für folgende(n) Vertragsstaat(en): AT, BE, CH, DE, DK, FR, GB, IT, LI, LU, NL, SE)

1. Ein Octapeptid mit biologischer Somatostatin-Aktivität, das an einer Aminosäure in 3-Stellung einen Tyrosinrest aufweist, wobei das Tyrosin in 3- bzw. 5-Stellung des Kohlenstoffs ein lodatom aufweist, oder ein pharmazeutisch nutzbares Salz davon.

2. Ein Octapeptid nach Anspruch 1 mit der Formel: 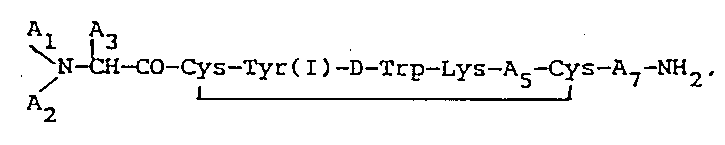 in der A₁ und A₂ jeweils unabhängig voneinander H, C₁₋₁₂ Alkyl, C₇₋₁₀ Phenylalkyl, R₁CO (wobei R₁ C₁₋₂₀ Alkyl, C₃₋₂₀ Alkenyl, C₃₋₂₀ Alkinyl, Phenyl, Naphthyl bzw. C₇₋₁₀ Phenylalkyl ist) oder R₂OCO (wobei R₂ C₁₋₁₀ Alkyl oder C₇₋₁₀ Phenylalkyl ist) bedeuten, sofern, wenn A₁ bzw. A₂ R₁CO oder R₂OCO ist, das jeweils andere H sein muß; A₃ bedeutet CH₂-A₆ (wobei A₆ Pentafluorophenyl, Naphthyl, Pyridyl, Phenyl oder o-, m- bzw. p-substituiertes Phenyl ist), wobei der Substituent ein Halogen, NH₂, NO₂, OH oder C₁₋₃ Alkyl ist; (I) bedeutet, daß Tyrosin in der 3- bzw. 5-Stellung des KJohlenstoffs am Phenylring iodiert wird; A₅ Thr, Ser, Phe, Val, α-Aminobuttersäure bzw. Ile bedeutet, vorausgesetzt, daß, wenn A₃ Phenyl ist, A₁ H und A₂ H ist, A₅ nicht Val sein kann; und A₇ Thr, Trp oder β-Nal bedeutet; oder ein pharmazeutisch nutzbares Salz davon.

3. Ein Octapeptid oder ein pharmazeutisch nutzbares Salz davon nach Anspruch 2, wobei 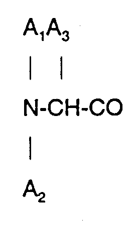 D-β-Naphthylalanin ist.

4. Ein Octapeptid oder ein pharmazeutisch nutzbares Salz davon nach Anspruch 2, wobei 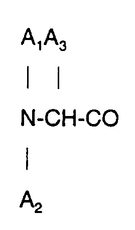 D-Phe und A₅ α-Aminobuttersäure ist.

5. Ein Octapeptid oder ein pharmazeutisch nutzbares Salz davon nach Anspruch 2, wobei R₁ CH₃ bzw. C₂H₅ ist.

6. Octapeptide der Formeln D-β-Nal-Cys-Tyr(I)-D-Trp-Lys-Val-Cys-Thr-NH₂, Pentafluoro-D-Phe-Cys-Tyr(I)-D-Trp-Lys-Val-Cys-Thr-NH₂, D-Phe-Cys-Tyr(I)-D-Trp-Lys-α-Aminobuttersäure-Cys-Thr-NH₂, N-Ac-D-β-Nal-Cys-Tyr(I)-D-Trp-Lys-Val-Cys-Thr-NH₂, D-β-Nal-Cys-Tyr(I)-D-Trp-Lys-Val-Cys-β-Nal-NH₂, D-Phe-Cys-Tyr(I)-D-Trp-Lys-Val-Cys-β-Nal-NH₂, D-β-Nal-Cys-Tyr(I)-D-Trp-Lys-α-Aminobuttersäure-Cys-Thr-NH₂; D-p-Cl-Phe-Cys-Tyr(I)-D-Trp-Lys-α-Aminobuttersäure-Cys-Thr-NH₂ oder Acetyl-D-p-C1-Phe-Cys-Tyr(I)-D-Trp-Cys-α-Aminobuttersäure-Cys-Thr-NH₂ oder pharmazeutisch nutzbare Salze davon.

7. Eine pharmazeutisch wirksame Zusammensetzung, die eine therapeutisch wirksame Menge eines Octapeptids oder eines pharmazeutisch nutzbaren Salzes davon nach einem der Ansprüche 1 bis 6 sowie eine pharmazeutisch nutzbare Trägersubstanz enthält.

8. Eine Zusammensetzung nach Anspruch 7, die in Form von Dragees, Tabletten oder Kapseln für die orale Verabreichung an erkrankte Menschen und Tiere oder in Form einer Flüssigkeit für die orale Verabreichung an erkrankte Menschen und Tiere vorliegt, oder die mit einer Substanz überzogen ist, die die Zusammensetzung vor der Säure im Magen des Menschen oder des Tiers solange schützt, bis sie unaufgelöst in den Darmtrakt des Patienten gelangt ist, oder in Form einer Creme, eines Gels, eines Sprays oder einer Salbe zum Auftragen auf die Haut von erkrankten Menschen oder Tieren, oder in Form einer Flüssigkeit zur nasalen Verabreichung als Tropfen oder Spray, oder in Form einer Flüssigkeit zur intravenösen, subkutanen, parenteralen oder intraperitonealen Anwendung beim Menschen und bei Tieren, oder in Form eines biologisch abbaubaren Retard-Präparates für die intramuskuläre Applikation beim Menschen und bei Tieren vorliegt.

9. Ein Octapeptid oder ein pharmazeutisch nutzbares Salz davon nach einem der Ansprüche 1 bis 6 zum Einsatz bei Behandlungen mit dem Ziel, die Freisetzung von Wachstumshormon, Insulin und Glucagon oder die exokrine Pankreassekretion zu vermindern bzw. zu hemmen oder zu unterbinden, bei der Behandlung von Diabetes, zum Schutz der Leber von Zirrhose- oder Hepatitispatienten, bei der Behandlung der Alzheimer-Krankheit, von Pilzvergiftungen oder von diabetes-bedingten Netzhauterkrankungen oder in der Krebstherapie, insbesondere zur Behandlung von wachstumshormon-bedingten Krebserkrankungen wie Knochenkrebs, Krebserkrankungen des Knorpelgewebes, Bauchspeicheldrüsenkrebs, Prostatakarzinom und Brustkrebs, von Akromegalie und ähnlichen, durch endokrine Mehrsekretion ausgelösten Erkrankungen, Ulcusblutungen bei Notfallpatienten oder Störungen des zentralen Nervensystems oder bei der Behandlung von Patienten mit Bauchspeicheldrüsenentzündung oder Durchfall.

10. Verwendung eines Octapeptids oder eines pharmazeutisch nutzbaren Salzes davon nach einem der Ansprüche 1 bis 6 bei der Herstellung eines Arzneimittels, das eingesetzt wird, um die Freisetzung von Wachstumshormon, Insulin und Glucagon und die exokrine Pankreassekretion zu vermindern bzw. zu hemmen oder zu unterbinden, sowie in der Diabetestherapie, zum Schutz der Leber vopn Zirrhose- und Hepatitispatienten, bei der Behandlung der Alzheimer-Krankheit, von Pilzvergiftungen oder diabetes-bedingten Netzhauterkrankungen oder in der Krebstherapie, insbesondere zur Behandlung von wachstumshormon-bedingten Krebserkrankungen wie Knochenkrebs, Krebserkrankungen des Knorpelgewebes, Bauchspeicheldrüsenkrebs, Prostatakarzinom und Brustkrebs, Akromegalie und ähnlichen, durch endokrine Mehrsekretion verursachten Erkrankungen, Ulcusblutungen bei Notfallpatienten und Störungen des zentralen Nervensystems oder bei der Behandlung von Patienten mit Bauchspeicheldrüsenentzündung oder Durchfall.

## Patentansprüche (Patentansprüche für folgende(n) Vertragsstaat(en): ES, GR)

1. Verfahren zur Herstellung eines neuen Octapeptids, das die Nutzung eines Octapeptids oder eines pharmazeutisch nutzbaren Salzes davon einschließt, wobei das besagte Octapeptid die biologische Aktivität von Somatostatin aufweist, einen Tyrosinrest in 3-Stellung an der Aminosäure hat und das besagte Tyrosin am Phenylring in 3- bzw. 5-Stellung des Kohlenstoffs iodiert ist.

2. Verfahren zur Herstellung eines neuen Octapeptids nach Anspruch 1, wobei das besagte Octapeptid folgende Formel hat: 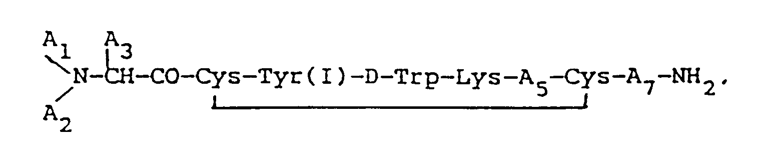 in der A₁ und A₂ jeweils unabhängig voneinander H, C₁₋₁₂ Alkyl, C₇₋₁₀ Phenylalkyl, R₁CO (wobei R₁ C₁₋₂₀ Alkyl, C₃₋₂₀ Alkenyl, C₃₋₂₀ Alkinyl, Phenyl, Naphthyl bzw. C₇₋₁₀ Phenylalkyl ist) oder R₂OCO (wobei R₂ C₁₋₁₀ Alkyl oder C₇₋₁₀ Phenylalkyl ist) bedeuten, sofern, wenn A₁ bzw. A₂ R₁CO oder R₂OCO ist, das jeweils andere H sein muß; A₃ bedeutet CH₂-A₆ (wobei A₆ Pentafluorophenyl, Naphthyl, Pyridyl, Phenyl oder o-, m- bzw. p-substituiertes Phenyl ist), wobei der Substituent ein Halogen, NH₂, NO₂, OH oder C₁₋₃ Alkyl ist; A₅ Thr, Ser, Phe, Val, α-Aminobuttersäure bzw. Ile bedeutet, vorausgesetzt, daß, wenn A₃ Phenyl ist, A₁ H und A₂ H ist, A₅ nicht Val sein kann; und A₇Thr, Trp oder β-Nal bedeutet.

3. Verfahren zur Herstellung eines Octapeptids nach Anspruch 2, wobei 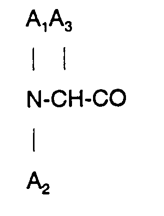 D-β-Naphthylalanin ist.

4. Verfahren zur Herstellung eines neuen Octapeptids nach Anspruch 2, wobei 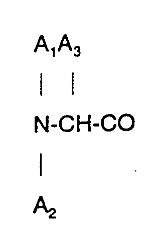 D-Phe und A₅ α-Aminobuttersäure ist.

5. Verfahren zur Herstellung eines neuen Octapeptids nach Anspruch 2, wobei R₁ CH₃ bzw. C₂H₅ ist.

6. Verfahren zur Herstellung eines neuen Octapeptids nach Anspruch 2, wobei das Octapeptid ausgewählt wird unter: D-β-Nal-Cys-Tyr-D-Trp-Lys-Val-Cys-Thr-NH₂, Pentafluoro-D-Phe-Cys-Tyr-D-Trp-Lys-Val-Cys-Thr-NH₂, D-Phe-Cys-Tyr-D-Trp-Lys-α-Aminobuttersäure-Cys-Thr-NH₂, N-Ac-D-β-Nal-Cys-Tyr-D-Trp-Lys-Val-Cys-Thr-NH₂, D-β-Nal-Cys-Tyr-D-Trp-Lys-Val-Cys-β-Nal-NH₂, D-Phe-Cys-Tyr-D-Trp-Lys-Val-Cys-β-Nal-NH₂, D-β-Nal-Cys-Tyr-D-Trp-Lys-α-Aminobuttersäure-Cys-Thr-NH₂; D-p-Cl-Phe-Cys-Tyr-D-Trp-Lys-α-Aminobuttersäure-Cys-Thr-NH₂ oder Acetyl-D-p-C1-Phe-Cys-Tyr-D-Trp-Cys-α-Aminobuttersäure-Cys-Thr-NH₂.

7. Verfahren zur Herstellung einer pharmazeutisch wirksamen Zusammensetzung, wobei das mittels eines in einem der Ansprüche 1 bis 6 genannten Verfahrens hergestellte Octapeptid-Produkt verwendet und mit einer pharmazeutisch nutzbaren Trägersubstanz vereinigt wird.

8. Verfahren zur Herstellung einer pharmazeutisch wirksamen Zusammensetzung unter Verwendung eines Octapeptids oder eines pharmazeutisch nutzbaren Salzes davon, wobei das Octapeptid die biologische Aktivität von Somatostatin aufweist, einen Tyrosinrest an der Aminosäure in 3-Stellung hat und das Tyrosin am Phenylring in 3- bzw. 5-Stellung des Kohlenstoffs iodiert ist, wobei das Octapeptid vorzugsweise folgende Formel hat: 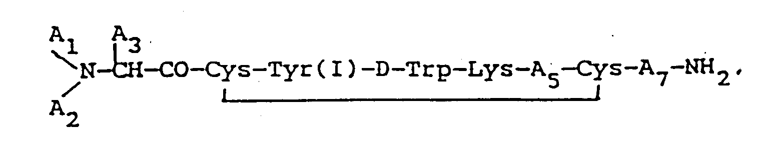 in der A₁ und A₂ jeweils unabhängig voneinander H, C₁₋₁₂ Alkyl, C₇₋₁₀ Phenylalkyl, R₁CO (wobei R₁ C₁₋₂₀ Alkyl, C₃₋₂₀ Alkenyl, C₃₋₂₀ Alkinyl, Phenyl, Naphthyl bzw. C₇₋₁₀ Phenylalkyl ist) oder R₂OCO (wobei R₂ C₁₋₁₀ Alkyl oder C₇₋₁₀ Phenylalkyl ist) bedeutet, sofern, wenn A₁ bzw. A₂ R₁CO oder R₂OCO ist, das jeweils andere H sein muß; A₃ bedeutet CH₂-A₆ (wobei A₆ Pentafluorophenyl, Naphthyl, Pyridyl, Phenyl oder o-, m- bzw. p-substituiertes Phenyl ist, wobei der Substituent ein Halogen, NH₂, NO₂, OH oder C₁₋₃ Alkyl ist); A₅ Thr, Ser, Phe, Val, α-Aminobuttersäure bzw. Ile bedeutet, vorausgesetzt, daß, wenn A₃ Phenyl ist, A₁ H und A₂ H ist, A₅ nicht Val sein kann; und A₇ Thr, Trp oder β-Nal bedeutet;
und vorzugsweise ausgewählt wird unter: D-β-Nal-Cys-Tyr-D-Trp-Lys-Val-Cys-Thr-NH₂, Pentafluoro-D-Phe-Cys-Tyr-D-Trp-Lys-Val-Cys-Thr-NH₂, D-Phe-Cys-Tyr-D-Trp-Lys-α-Aminobuttersäure-Cys-Thr-NH₂, N-Ac-D-β-Nal-Cys-Tyr-D-Trp-Lys-Val-Cys-Thr-NH₂, D-β-Nal-Cys-Tyr-D-Trp-Lys-Val-Cys-β-Nal-NH₂, D-Phe-Cys-Tyr-D-Trp-Lys-Val-Cys-β-NaI-NH₂, D-β-Nal-Cys-Tyr-D-Trp-Lys-α-Aminobuttersäure-Cys-Thr-NH₂, D-p-Cl-Phe-Cys-Tyr-D-Trp-Lys-α-Aminobuttersäure-Cys-Thr-NH₂ oder Acetyl-D-p-C1-Phe-Cys-Tyr-D-Trp-Cys-α-Aminobuttersäure-Cys-Thr-NH₂,
und Mischen des Octapeptids mit einer pharmazeutisch nutzbaren Trägersubstanz zur Gewinnung der pharmazeutischen Zusammensetzung.

9. Verwendung eines Octapeptids oder eines pharmazeutisch nutzbaren Salzes davon, wobei das besagte Octapeptid die biologische Aktivität von Somatostatin aufweist, einen Tyrosinrest an der Aminosäure in 3-Stellung hat und das besagte Tyrosin am Phenylring in der 3- bzw. 5-Stellung des Kohlenstoffs iodiert ist, wobei das besagte Octapeptid vorzugsweise folgende Formel hat: 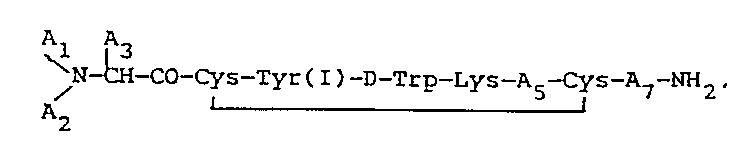 in der A₁ und A₂ jeweils unabhängig voneinander H, C₁₋₁₂ Alkyl, C₇₋₁₀ Phenylalkyl, R₁CO (wobei R₁ C₁₋₂₀ Alkyl, C₃₋₂₀ Alkenyl, C₃₋₂₀ Alkinyl, Phenyl, Naphthyl bzw. C₇₋₁₀ Phenylalkyl ist) oder R₂OCO (wobei R₂ C₁₋₁₀ Alkyl oder C₇₋₁₀ Phenylalkyl ist) bedeuten, sofern, wenn A₁ bzw. A₂ R₁CO oder R₂OCO ist, das jeweils andere H sein muß; A₃ CH₂-A₆ bedeutet (wobei A₆ Pentafluorophenyl, Naphthyl, Pyridyl, Phenyl oder o-, m- bzw. p-substituiertes Phenyl ist, wobei der Substituent ein Halogen, NH₂, NO₂, OH oder C₁₋₃ Alkyl ist); A₅ Thr, Ser, Phe, Val, α-Aminobuttersäure bzw. Ile bedeutet, vorausgesetzt, daß, wenn A₃ Phenyl ist, A₁ H und A₂ H ist, A₅ nicht Val sein kann; und A₇ Thr, Trp oder β-Nal bedeutet
und vorzugsweise ausgewählt wird unter: D-β-Nal-Cys-Tyr-D-Trp-Lys-Val-Cys-Thr-NH₂, Pentafluoro-D-Phe-Cys-Tyr-D-Trp-Lys-Val-Cys-Thr-NH₂, D-Phe-Cys-Tyr-D-Trp-Lys-α-Aminobuttersäure-Cys-Thr-NH₂, N-Ac-D-β-Nal-Cys-Tyr-D-Trp-Lys-Val-Cys-Thr-NH₂, D-β-Nal-Cys-Tyr-D-Trp-Lys-Val-Cys-β-Nal-NH₂, D-Phe-Cys-Tyr-D-Trp-Lys-Val-Cys-β-Nal-NH₂, D-β-Nal-Cys-Tyr-D-Trp-Lys-α-Aminobuttersäure-Cys-Thr-NH₂, D-p-Cl-Phe-Cys-Tyr-D-Trp-Lys-α-Aminobuttersäure-Cys-Thr-NH₂ oder Acetyl-D-p-C1-Phe-Cys-Tyr-D-Trp-Cys-α-Aminobuttersäure-Cys-Thr-NH₂,
für die Herstellung eines Medikaments, das eingesetzt wird, um die Freisetzung von Wachstumshormon, Insulin oder Glucagon bzw. die exokrine Pankreassekretion zu vermindern bzw. zu hemmen oder zu unterbinden, sowie in der Diabetestherapie, zum Schutz der Leber von Zirrhose- oder Hepatitispatienten, bei der Behandlung der Alzheimer-Krankheit, von Pilzvergiftungen und diabetes-bedingten Netzhauterkrankungen sowie in der Krebstherapie, insbesondere von wachstumshormon-bedingten Krebserkrankungen wie Knochenkrebs, Krebserkrankungen des Knorpelgewebes, Bauchspeicheldrüsenkrebs, Prostatakarzinom und Brustkrebs, der Therapie von Akromegalie und ähnlichen durch endokrine Mehrsekretion ausgelösten Erkrankungen, zur Behandlung von Ulcusblutungen bei Notfallpatienten, von Störungen des zentralen Nervensystems und bei der Behandlung von Patienten mit Bauchspeicheldrüsenentzündung oder Durchfall.

## Revendications (Revendications pour l'(les) Etat(s) contractant(s) suivant(s): AT, BE, CH, DE, DK, FR, GB, IT, LI, LU, NL, SE)

1. Octapeptide ayant une activité biologique de somatostatine, comportant en position 3 des aminoacides un reste tyrosine, ladite tyrosine comprenant un atome d'iode sur ses positions de carbone 3 ou 5, ou un sel pharmaceutiquement acceptable d'un tel octapeptide.

2. Octapeptide selon la revendication 1, caractérisé en ce qu'il a la formule : 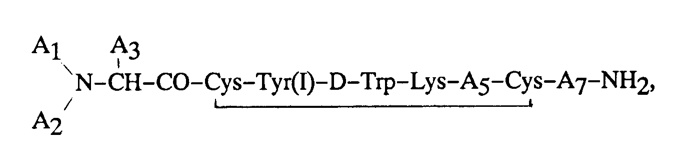 dans laquelle chaque symbole A₁ et A₂ représente indépendamment, H, un groupe alkyle en C₁-C₁₂, phénylalkyle en C₇-C₁₀, R₁CO (dans lequel R₁ représente un groupe alkyle en C₁-C₂₀, alcényle en C₃-C₂₀, alcynyle en C₃-C₂₀, phényle, naphtyle ou phénylalkyle en C₇-C₁₀), ou R₂OCO (dans lequel R₂ représente un groupe alkyle en C₁-C₁₀ ou phénylalkyle en C₇-C₁₀), à la condition que, lorsque l'un des symboles A₁ ou A₂ représente R₁CO ou R₂OCO, l'autre doit représenter H ; A₃ représente CH₂-A₆ (formule dans laquelle A₆ représente un groupe pentafluorophényle, naphtyle, pyridyle, phényle ou phényle substitué en ortho, en méta ou en para, ledit substituant étant un atome d'halogène, un reste NH₂, NO₂, OH ou alkyle en C₁-C₃) ; (I) indique que la tyrosine est iodée sur le noyau phényle sur le carbone en position 3 ou 5 ; A₅ représente Thr, Ser, Phe, Val, l'acide α-aminobutyrique ou Ile, à la condition que, lorsque A₃ représente un groupe phényle, A₁ représente H et A₂ représente H, A₅ ne peut pas représenter un reste Val ; et A₇ représente Thr, Trp ou β-Nal ; ou un sel pharmaceutiquement acceptable de cet octapeptide.

3. Octapeptide, ou sel pharmaceutiquement acceptable de ce omposé selon la revendication 2, dans lequel le groupe de formule 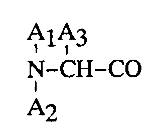 représente un groupe D-β-naphtylalanine.

4. Octapeptide, ou un sel pharmaceutiquement acceptable de cet octapeptide selon la revendication 2, dans lequel 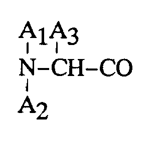 représente un reste D-Phe et A₃ représente de l'acide α-aminobutyrique.

5. Octapeptide, ou un sel pharmaceutiquement acceptable de cet octapeptide selon la revendication 2, dans lequel R₁ représente CH₃ ou C₂H₅.

6. Octapeptide répondant aux formules de la D-β-Nal-Cys-Tyr(I)-D-Trp-Lys-Val-Cys-Thr-NH₂; de la pentafluoro-D-Phe-Cys-Tyr(I)-D-Trp-Lys-Val-Cys-Thr-NH₂ ; de la D-Phe-Cys-Tyr(I)-D-Trp-Lys-(acide α-aminobutyrique)-Cys-Thr, NH₂ ; de la N-Ac-D-β-Nal-Cys-Tyr(I)-D-Trp-Lys-Val-Cys-Thr-NH₂ ; de la D-β-Nal-Cys-Tyr(I)-D-Trp-Lys-Val-Cys-β-Nal-NH₂ ; la D-Phe-Cys-Tyr-(I)-D-Trp-Lys-Val-Cys-β-Nal-NH₂ ; la D-β-Nal-Cys-Tyr(I)-D-Trp-Lys-(acide α-aminobutyrique)-Cys-Thr-NH₂ ; de la D-p-Cl-Phe-Cys-Tyr(I)-D-Trp-Lys-(acide α-aminobutyrique)-Cys-Thr-NH₂ ; ou de l'acétyl-D-p-Cl-Phe-Cys-Tyr(I)-D-Trp-Cys-(acide α-aminobutyrique)-Cys-Thr-NH₂ ou leurs sels pharmaceutiquement acceptables.

7. Composition pharmaceutique comprenant une quantité thérapeutiquement efficace d'un octapeptide ou d'un sel pharmaceutiquement acceptable de cet octapeptide, tel que revendiqué dans l'une quelconque des revendications 1 à 6, avec une substance constituant un excipient pharmaceutiquement acceptable.

8. Composition selon la revendication 7, dans laquelle ladite composition est sous forme d'une pilule, d'un comprimé ou d'une capsule ou gelule pour administration par voie orale à un être humain ou un patient animal, ou sous forme d'un liquide pour administration par voie orale à un patient humain ou animal, ou est enrobée par une substance capable de protéger ladite composition contre l'acide gastrique dans l'estomac d'un patient humain ou animal pendant une période de temps suffisante pour permettre à ladite composition de passer, sans être désagrégée, dans l'intestin grêle dudit patient, ou qui est sous forme d'une crème, d'un gel, d'un produit à pulvériser ("spray") ou d'un onguent ou pommade à appliquer sur la peau d'un patient humain ou animal, ou qui est sous forme d'un liquide capable d'être administré par voie nasale sous forme de gouttes ou de pulvérisation ("spray") à un patient humain ou animal, ou qui est sous forme d'un liquide pour administration par voie intraveineuse, sous-cutanée, parentérale ou intrapéritonéale à un patient humain ou animal ou qui est sous forme d'une composition biodégradable, à libération soutenue pour administration intramusculaire à un patient humain ou animal.

9. Octapeptide, ou son sel pharmaceutiquement acceptable selon l'une quelconque des revendications 1 à 6, destiné à server dans un traitement visant à diminuer ou à inhiber le dégagement de libération, ou à exercer un effet antagoniste contre, une hormone de croissance, l'insuline, le glucagon ou une sécrétion exocrine du pancréas, pour traiter le diabète, pour protéger le fois de patients souffrant de cirrhose ou d'une hépatite, pour traiter la maladie d'Alzheimer, un empoisonnement par des champignons ou une rétinopathie liée à du diabète, ou pour le traitement d'un cancer, en particulier d'un cancer dépendant de l'hormone de croissance comme un cancer de l'os, du cartilage, du pancréas, de la prostate ou des seins, ou de la poitrine, pour traiter une acromélagie et des états d'hypersécrétion endocrine apparentés, des ulcères qui saignent chez les patients admis en urgence, et des troubles du système nerveux central, ou pour traiter des patients souffrant d'une pancréatite ou de diarrhée.

10. Utilisation d'un octapeptide ou d'un sel pharmaceutiquement acceptable de cet octapeptide selon l'une quelconque des revendications 1 à 6, pour la fabrication d'un médicament destiné à servir dans un traitement visant à diminuer ou à inhiber le dégagement de libération, ou à exercer un effet antagoniste de l'effet de l'hormone de croissance, de l'insuline, du glucagon ou d'une sécrétion exocrine du pancréas, pour traiter le diabète, pour protéger le foie de patients souffrant de cirrhose ou d'hépatite, pour traiter la maladie d'Alzheimer, un empoisonnement par des champignons ou une rétinopathie liée à du diabète, ou pour traiter un cancer, en particulier un cancer dépendant de l'hormone de croissance comme un cancer de l'os, du cartilage, du pancréas, de la protate ou du sein ou de la poitrine, pour traiter l'acromélagie, et des états d'hypersécrétion endocrine apparentés, des ulcères qui saignent chez des patients admis en urgence, des troubles du système nerveux central ou de patients souffrant de pancréatite ou de diarrhée.

## Revendications (Revendications pour l'(les) Etat(s) contractant(s) suivant(s): ES, GR)

1. Procédé pour préparer un nouvel octapeptide, comprenant la mise en oeuvre d'un octapeptide, ou d'un sel pharmaceutiquement acceptable de celui-ci, ledit octapeptide ayant l'activité biologique de la somatostatine, ledit octapeptide comportant un reste tyrosine sur la position de l'aminoacide 3 et à ioder ladite tyrosine sur un carbone en position 3 ou 5 du noyau phényle.

2. Procédé pour préparer un nouvel octapeptide selon la revendication 1, dans lequel ledit octapeptide a pour formule : 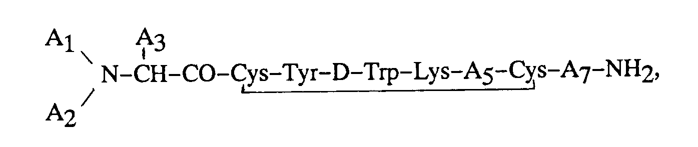 dans laquelle chaque symbole A₁ et A₂ représentent, indépendamment, un atome de H, un groupe alkyle en C₁-C₁₂, phénylalkyle en C₇-C₁₀, R₁CO (dans lequel R₁ représente un reste alkyle en C₁-C₂₀, alcényle en C₃-C₂₀, alcynyle en C₃-C₂₀, phényle, naphtyle ou phénylalkyle en C₇-C₁₀), ou R₂OCO (formule dans laquelle R₂ représente un reste alkyle en C₁-C₁₀ ou phénylalkyle en C₇-C₁₀), à la condition que, lorsque l'un des symboles A₁ ou A₂ représente R₁CO ou R₂OCO, l'autre symbole doit représenter H ; A₃ représente CH₂-A₆ (formule dans laquelle A₆ représente un reste pentafluorophényle, naphtyle, pyridyle, phényle ou phényle substitué en ortho, en méta ou en para, ledit substituant étant un atome d'halogène, ou un reste NH₂, NO₂, OH ou alkyle en C₁-C₃);
A₅ représente Thr, Ser, Phe, Val, l'acide α-aminobutyrique ou Ile, à la condition que, lorsque A₃ représente un groupe phényle, que A₁ représente H et A₂ représente H, A₅ ne peut pas représenter un reste Val ; et A₇ représente Thr, Trp ou β-Nal.

3. Procédé de préparation d'un octapeptide selon la revendication 2, dans lequel 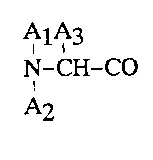 est un reste D-β-naphtylalanine.

4. Procédé de préparation d'un nouvel octapeptide selon la revendication 2, dans lequel 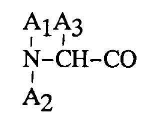 représente D-Phe et A₅ représente l'acide α-aminobutyrique.

5. Procédé de préparation d'un nouvel octapeptide selon la revendication 2, dans lequel R₁ représente CH₃ ou C₂H₅.

6. Procédé de préparation d'un nouvel octapeptide selon la revendication 2, dans lequel ledit octapeptide est choisi parmi la D-β-Nal-Cys-Tyr-D-Trp-Lys-Val-Cys-Thr-NH₂ ; la pentafluoro-D-Phe-Cys-Tyr-D-Trp-Lys-Val-Cys-Thr-NH₂ ; la D-Phe-Cys-Tyr-D-Trp-Lys-(acide α-aminobutyrique)-Cys-Thr-NH₂ ; la N-Ac-D-β-Nal-Cys-Tyr-D-Trp-Lys-Val-Cys-Thr-NH₂ ; la D-β-Nal-Cys-Tyr-D-Trp-Lys-Val-Cys-β-Nal-NH₂ ; la D-Phe-Cys-Tyr-D-Trp-Lys-Val-Cys-β-Nal-NH₂ ; la D-β-Nal-Cys-Tyr-D-Trp-Lys-(acide α-aminobutyrique)-Cys-Thr-NH₂ ; la D-p-Cl-Phe-Cys-Tyr-D-Trp-Lys-(acide α-aminobutyrique)-Cys-Thr-NH₂ ; ou l'acétyl-D-p-Cl-Phe-Cys-Tyr-D-Trp-Cys-(acide α-aminobutyrique)-Cys-Thr-NH₂.

7. Procédé de préparation d'une composition pharmaceutique, comprenant la mise en oeuvre de l'octapeptide, produit du procédé selon l'une quelconque des revendications 1 à 6, et la combinaison dudit produit avec une substance à rôle d'excipient pharmaceutique.

8. Procédé pour préparer une composition pharmaceutique, comprenant la mise en oeuvre d'un octapeptide ou d'un sel pharmaceutiquement acceptable de cet octapeptide, ledit octapeptide ayant l'activité biologique de la somatostatine, comportant un reste tyrosine sur la position de l'aminoacide 3 et ladite tyrosine étant iodée sur le noyau phényle, sur le carbone en position 3 ou en position 5 de ce noyau, ledit octapeptide ayant de préférence la formule : 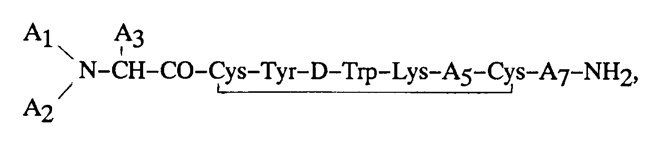 dans laquelle chaque symbole A₁ et A₂ représente indépendamment, un atome de H, un groupe alkyle en C₁-C₁₂, phénylalkyle en C₇-C₁₀, R₁CO (formule dans laquelle R₁ représente un reste alkyle en C₁-C₂₀, alcényle en C₃-C₂₀, alcynyle en C₃-C₂₀, phényle, naphtyle ou phénylalkyle en C₇-C₁₀), ou R₂OCO (formule dans laquelle R₂ représente un reste alkyle en C₁-C₁₀ ou phénylalkyle en C₇-C₁₀), à la condition que, lorsque l'un des symboles A₁ ou A₂ représente R₁CO ou R₂OCO, l'autre symbole doit représenter H ; A₃ représente CH₂-A₆ (formule dans laquelle A₆ représente un reste pentafluorophényle, naphtyle, pyridyle, phényle ou bien phényle substitué en ortho, en méta ou en para, ledit substituant étant un atome d'halogène, ou un reste NH₂, NO₂, OH ou alkyle en C₁-C₃) ;
A₅ représente un reste Thr, Ser, Phe, Val, acide α-aminobutyrique ou Ile, à la condition que, lorsque A₃ représente un reste phényle, que A₁ représente H et A₂ représente H, le symbole A₅ ne peut pas représenter un reste Val ; et A₇ représente Thr, Trp ou β-Nal ;
et, ce composé étant encore mieux choisi parmi
la D-β-Nal-Cys-Tyr-D-Trp-Lys-Val-Cys-Thr-NH₂ ; la pentafluoro-D-Phe-Cys-Tyr-D-Trp-Lys-Val-Cys-Thr-NH₂ ; la D-Phe-Cys-Tyr-D-Trp-Lys-(acide α-aminobutyrique)-Cys-Thr-NH₂ ; la N-Ac-D-β-Nal-Cys-Tyr-D-Trp-Lys-Val-Cys-Thr-NH₂ ; la D-β-Nal-Cys-Tyr-D-Trp-Lys-Val-Cys-β-Nal-NH₂ ; la D-Phe-Cys-Tyr-D-Trp-Lys-Val-Cys-β-Nal-NH₂ ; la D-β-Nal-Cys-Tyr-D-Trp-Lys-(acide α-aminobutyrique)-Cys-Thr-NH₂ ; la D-p-Cl-Phe-Cys-Tyr-D-Trp-Lys-(acide α-aminobutyrique)-Cys-Thr-NH₂ ; ou l'acétyl-D-p-Cl-Phe-Cys-Tyr-D-Trp-Cys-(acide α-aminobutyrique)-Cys-Thr-NH₂,
et le mélangeage dudit octapeptide avec un excipient, matière pharmaceutiquement acceptable, pour former ladite composition pharmaceutique.

9. Utilisation d'un octapeptide, ou d'un sel pharmaceutiquement acceptable de cet octapeptide, ledit octapeptide ayant l'activité biologique de la somatostatine, comportant un reste tyrosine sur la position de l'aminoacide 3 et ladite tyrosine étant iodée sur ses carbones en position 3 ou 5 du noyau phényle, ledit octapeptide ayant de préférence la formule : 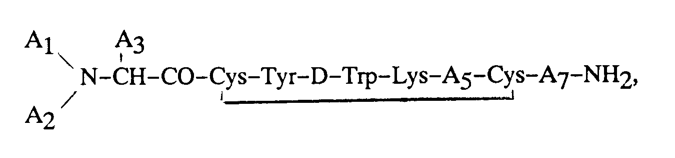 dans laquelle chaque symbole A₁ et A₂ représente indépendamment, un atome de H, un groupe alkyle en C₁-C₁₂, phénylalkyle en C₇-C₁₀, R₁CO (formule dans laquelle R₁ représente un reste alkyle en C₁-C₂₀, alcényle en C₃-C₂₀, alcynyle en C₃-C₂₀, phényle, naphtyle ou phénylalkyle en C₇-C₁₀), ou R₂OCO (formule dans laquelle R₂ représente un reste alkyle en C₁-C₁₀ ou phénylalkyle en C₇-C₁₀), à la condition que, lorsque l'un des symboles A₁ ou A₂ représente R₁CO ou R₂OCO, l'autre symbole doit représenter H ; A₃ représente CH₂-A₆ (formule dans laquelle A₆ représente un reste pentafluorophényle, naphtyle, pyridyle, phényle ou bien phényle substitué en ortho, en méta ou en para, ledit substituant étant un atome d'halogène, ou un reste NH₂, NO₂, OH ou alkyle en C₁-C₃) ;
A₅ représente un reste Thr, Ser, Phe, Val, acide α-aminobutyrique ou Ile, à la condition que, lorsque A₃ représente un reste phényle, que A₁ représente H et A₂ représente H, le symbole A₅ ne peut pas représenter un reste Val ; et A₇ représente Thr, Trp ou β-Nal ;
et étant encore mieux choisi parmi
la D-β-Nal-Cys-Tyr-D-Trp-Lys-Val-Cys-Thr-NH₂ ; la pentafluoro-D-Phe-Cys-Tyr-D-Trp-Lys-Val-Cys-Thr-NH₂ ; la D-Phe-Cys-Tyr-D-Trp-Lys-(acide α-aminobutyrique)-Cys-Thr-NH₂ ; la N-Ac-D-β-Nal-Cys-Tyr-D-Trp-Lys-Val-Cys-Thr-NH₂ ; la D-β-Nal-Cys-Tyr-D-Trp-Lys-Val-Cys-β-Nal-NH₂ ; la D-Phe-Cys-Tyr-D-Trp-Lys-Val-Cys-β-Nal-NH₂ ; la D-β-Nal-Cys-Tyr-D-Trp-Lys-(acide α-aminobutyrique)-Cys-Thr-NH₂ ; la D-p-Cl-Phe-Cys-Tyr-D-Trp-Lys-(acide α-aminobutyrique)-Cys-Thr-NH₂ ; ou l'acétyl-D-p-Cl-Phe-Cys-Tyr-D-Trp-Cys-(acide α-aminobutyrique)-Cys-Thr-NH₂,
pour la fabrication d'un médicament destiné à servir dans un traitement pour diminuer ou inhiber le dégagement de libération ou pour exercer un effet antagoniste contre l'effet de l'hormone de la croissance, l'insuline, le glucagon, ou une sécrétion exocrine du pancréas, pour le traitement du diabète pour protéger le foie de patients souffrant de cirrhose ou d'hépatite, pour traiter une maladie d'Alzheimer, un empoisonnement par des champignons, ou une rétinopathie liée à du diabète, ou pour traiter un cancer, en particulier un cancer dépendant de l'hormone de croissance comme un cancer de l'os, du cartilage, du pancréas, de la prostate ou du sein ou de la poitrine, pour traiter l'acromégalie et des états d'hypersécrétion endocrine apparentés, des ulcères qui saignent chez des patients admis en urgence, des troubles du système nerveux central, ou des patients souffrant de pancréatite ou de diarrhée.
